# EUROPÄISCHE PATENTSCHRIFT

(11) **EP 0 683 645 B1**
(45) Veröffentlichungstag und Bekanntmachung des Hinweises auf die Patenterteilung: **11.06.1997**
(21) Anmeldenummer: 94906792.0
(22) Anmeldetag: 09.02.1994
(51) Int. Cl.: A61B 17/43

(54) **EINRICHTUNG ZUR GEWINNUNG VON SAMENZELLEN AUS SAMENFLÜSSIGKEIT**
DEVICE FOR REMOVING SPERM CELLS FROM SEMINAL FLUID
DISPOSITIF PERMETTANT D'EXTRAIRE DES SPERMATOZOIDES CONTENUS DANS DU LIQUIDE SEMINAL

(30) Priorität: 09.02.1993 AT 225/93; 08.10.1993 AT 2019/93
(43) Veröffentlichungstag der Anmeldung: 29.11.1995
(73) Patentinhaber: ZECH, Josef, A-6020 Innsbruck (AT)
(72) Erfinder: ZECH, Josef, A-6020 Innsbruck (AT)
(74) Vertreter: Hofinger, Engelbert, DDr.
(86) Internationale Anmeldenummer: AT9400014
(87) Internationale Veröffentlichungsnummer: WO9417742

(56) Entgegenhaltungen:
- FR-A- 2 539 628
- GB-A- 2 220 003

## Beschreibung

Die Erfindung bezieht sich auf eine Einrichtung zur Gewinnung von Samenzellen aus Samenflüssigkeit, mit einem ersten Gefäß zur Aufnahme der Samenflüssigkeit und einem zweiten Gefäß zur Aufnahme der aus der Samenflüssigkeit abgetrennten Samenzellen, wobei die beiden Gefäße über eine Flüssigkeitsbrücke verbunden sind, wenn die Samenflüssigkeit mit Nährlösung überdeckt ist.

Bei der künstlichen Befruchtung werden üblicherweise die Samenzellen aus der Samenflüssigkeit abgetrennt, bevor sie in-vitro oder in-utero mit einer Eizelle in Kontakt gebracht werden. Durch diesen Trennschritt kann es zur Beschädigung der Samenzellen kommen.

In der FR-PS 2 539 628 wurde bereits eine Einrichtung zur In-Vitro-Fertilisation mit einem Behälter für die Eizelle und einem Behälter für Samenflüssigkeit vorgeschlagen, wobei die Behälter kommunizieren, wenn Eizelle und Samenflüssigkeit mit Nährlösung überdeckt sind. Das Problem bei dieser Einrichtung liegt darin, daß die Flüssigkeitsbrücke zwischen den beiden Behältern einfach durch jenen Teil der Nährflüssigkeit gebildet wird, welcher über den oberen Rand des inneren Behälters (welcher die Eizelle aufnimmt) übersteht. Besonders wenn die Behälter, etwa zum Mikroskopieren, bewegt werden, schwappt die Nährflüssigkeit zwischen den Teilbehältern über, sodaß nicht die Beweglichkeit der Spermien, sondern äußere Einflüsse bestimmen, welche der Samenzellen zur Befruchtung führt. Mehrfachbefruchtungen sind nicht auszuschließen.

Die Erfindung strebt eine möglichst große Annäherung an die natürlichen Verhältnisse an. Unter Vermeidung von schädlichen Abtrennungsschritten, wie Zentrifugieren, sollen also bevorzugt die beweglichsten Spermien zur Befruchtung zugelassen werden.

Erfindungsgemäß ist bei einer Einrichtung der eingangs genannten Art vorgesehen, daß der Querschnitt der Flüssigkeitsbrücke durch feste Wände begrenzt ist, wobei das zweite Gefäß innerhalb des ersten Gefäßes angeordnet ist und ein Brückenteil vorgesehen ist, welches den Rand des zweiten Gefäßes U-förmig übergreift und einen inneren sowie einen äußeren Abschnitt besitzt, der in das zweite bzw. in das erste Gefäß ragt.

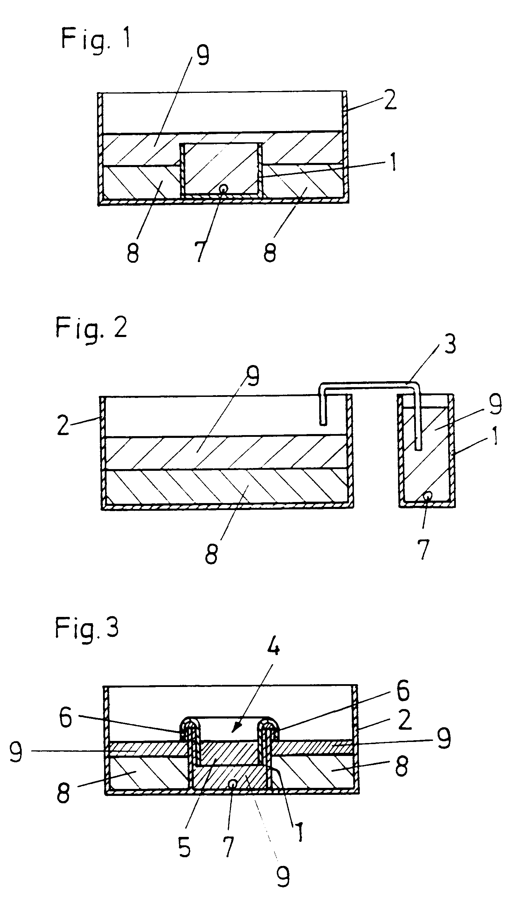

In der Zeichnung bezieht sich Fig. 1 auf den Stand der Technik, Fig. 2, 3, 4 und 5 sind Ausführungsbeispiele der Erfindung.

Die bekannte Einrichtung nach Fig. 1 besteht aus einem Gefäß 2 zur Aufnahme von Samenflüssigkeit, in welchem ein Gefäß 1 zur Aufnahme abgetrennter Samenzellen angeordnet ist. Überdeckt man die Samenflüssigkeit 8 mit Nährlösung 9, so gelangen einzelne Samenzellen in die Nährlösung. Der Transport der Samenzellen zur Eizelle 7 erfolgt jedoch nicht ausschließlich durch die Beweglichkeit der Samenzellen, sondern auch durch die makroskopische Strömung der Nährlösung.

Gemäß der Erfindung sind die Gefäße 1 und 2 lediglich durch eine Leitung miteinander verbunden, in welcher keine wesentliche Strömung stattfindet. Zur Ausbildung dieser Leitung kann ein innen hohler Brückenteil zwischen den beiden Gefäßen 1 und 2 verwendet werden, der beim Ausführungsbeispiel nach Fig. 2 als Kapillarröhrchen ausgebildet ist. Die Nährlösung 9 im Gefäß 1 wird hier so hoch eingefüllt, bis durch Kapillarwirkung das ganze Röhrchen 3 gefüllt ist. Erhöht man anschließend das Niveau der Nährlösung 9 im Gefäß 2, so können sich wohl einzelne Spermien durch das Röhrchen 3 bewegen, es erfolgt jedoch keine Bewegung der Nährlösung als solcher zwischen den beiden Gefäßen.

Eine besonders zweckmäßige Art, eine Kapillarleitung herzustellen, zeigt Fig. 3: hier sind die beiden Gefäße 1 und 2 konzentrisch ineinander angeordnet. In das Gefäß 1 wird Nährflüssigkeit 9 gefüllt, in das Gefäß 2 wird Samenflüssigkeit 8 eingebracht. Anschließend wird ein den Rand des Gefäßes 1 U-förmig übergreifender Brückenteil 4 lose auf das Gefäß 1 aufgelegt. Der in das zweite Gefäß 1 ragende innere Abschnitt 5 dieses Brückenteils 4 ist länger als der in das erste Gefäß 2 ragende äußere Abschnitt 6. Durch Kapillarwirkung steigt die Nährflüssigkeit im Spalt zwischen Brückenteil 4 und Gefäß 1 nach oben und überschichtet langsam die Samenflüssigkeit 8, bis der Flüssigkeitsstand an den Gefäßen 1 und 2 die gleiche Höhe erreicht hat.

Die dargestellte Einrichtung dient zur In-Vitro-Fertilisation, wenn im Gefäß 1, welches zur Aufnahme der abgetrennten Samenzellen dient, Eizellen 7 angeordnet werden. Soll die Befruchtung hingegen in-utero erfolgen, wird im Gefäß 1 keine Eizelle angeordnet und es erfolgt dort lediglich eine Sammlung gereinigter Samenzellen.

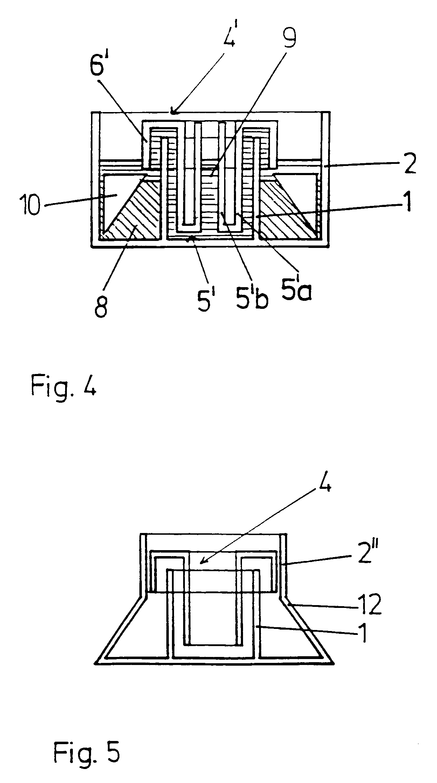

Im Ausführungsbeispiel nach Fig. 4 sind die beiden Gefäße 1 und 2 wiederum konzentrisch ineinander angeordnet. Der innere Abschnitt 5' des Brückenteils 4' besteht hier aus einem im wesentlichen bis zum Boden des Gefäßes 1 ragenden Teilabschnitt 5'a und einem innen anschließenden Teilabschnitt 5'b, der ein im Vergleich zum Volumen des Gefäßes 1 kleineres Volumen zur Aufnahme von Nährlösung 9 eingrenzt. Durch die Verringerung des Volumens der Nährlösung bei gleichbleibender Ringfläche der Flüssigkeitsbrücke wird schneller eine ausreichend hohe Konzentration von Spermien in der Nährlösung 9 erhalten. Die Wegstrecke, die die Spermien zurückzulegen haben, ist bei dieser Form des Brückenteils etwas größer als im Ausführungsbeispiel nach Fig. 3.

In das Gefäß 2 der Fig. 4 ist ein Glasring 10 eingelegt, der einen zweckmäßigen Raum zur Aufnahme der Samenflüssigkeit 8 ausbildet, der sich nach oben in Richtung zum äußeren Abschnitt 6' des Brückenteiles 4' verjüngt.

Ein solcherart ausgebildeter Raum zur Aufnahme der Samenflüssigkeit kann auch durch die Verwendung eines Gefäßes 2'' der in Fig. 5 gezeigten Form, mit einem sich in Richtung zum zweiten Gefäß 1 neigenden Abschnitt 12, erreicht werden.

In zahlreichen Versuchen wurde festgestellt, daß mit der dargestellten Einrichtung auch aus stark kontaminierter Flüssigkeit reine Samenzellen gewonnen werden können. Darin liegt ein wesentlicher Vorteil gegenüber bekannten Verfahren, bei welchen Samenflüssigkeit mit einer Nährlösung überdeckt und die Nährlösung nach Anreicherung mit Samenzellen abgesaugt wird (swim up- Methode). Es wird vermutet, daß das bessere Ergebnis darauf zurückzuführen ist, daß gemäß der Erfindung die Spermien einen Weg zurückzulegen haben, welcher wesentlich größer ist als jene Strecke, die in vergleichbaren Zeiten (Größenordnung 1 Stunde) von Bakterien aufgrund von Diffusionsvorgängen zurückgelegt werden.

## Patentansprüche

1. Einrichtung zur Gewinnung von Samenzellen aus Samenflüssigkeit (8), mit einem ersten Gefäß (2) zur Aufnahme der Samenflüssigkeit (8) und einem zweiten Gefäß (1) zur Aufnahme der aus der Samenflüssigkeit (8) abgetrennten Samenzellen, wobei die beiden Gefäße über eine Flüssigkeitsbrücke verbunden sind, wenn die Samenflüssigkeit (8) mit Nährlösung überdeckt ist, dadurch gekennzeichnet, daß der Querschnitt der Flüssigkeitsbrücke durch feste Wände begrenzt ist, wobei das zweite Gefäß (1) innerhalb des ersten Gefäßes (2) angeordnet ist und ein Brückenteil (4) vorgesehen ist, welches den Rand des zweiten Gefäßes (1) U-förmig übergreift und einen inneren (5, 5') sowie einen äußeren Abschnitt (6) besitzt, der in das zweite (1) bzw. in das erste Gefäß (2) ragt.

2. Einrichtung nach Anspruch 1, dadurch gekennzeichnet, daß der innere Abschnitt (5) des Brückenteils (4) länger ist als der in das erste Gefäß (2) ragende äußere Abschnitt (6).

3. Einrichtung nach Anspruch 1 oder Anspruch 2, dadurch gekennzeichnet, daß der innere Abschnitt (5') des Brückenteils (4') sich aus einem bis fast zum Boden des zweiten Gefäßes (1) ragenden Teilabschnitt (5'a) und einen innen anschließenden Teilabschnitt (5'b), der ein im Vergleich zum Volumen des zweiten Gefäßes (1) kleineres Volumen zur Aufnahme von Nährlösung (9) eingrenzt, zusammensetzt.

4. Einrichtung nach einem der Ansprüche 1 bis 3, dadurch gekennzeichnet, daß die vom zweiten Gefäß (1) abgelegene Wand des ersten Gefäßes (2'') einen sich in Richtung zum zweiten Gefäß (1) neigenden Abschnitt (12) aufweist, wobei ein sich nach oben in Richtung zur Flüssigkeitsbrücke verjüngender Raum zur Aufnahme der Samenflüssigkeit ausgebildet wird.

5. Einrichtung nach einem der Ansprüche 1 bis 3, dadurch gekennzeichnet, daß ein in das erste Gefäß (2) einlegbarer Glasteil (10) einen sich nach oben in Richtung zur Flüssigkeitsbrücke verjüngenden Raum zur Aufnahme der Samenflüssigkeit (8) ausbildet.

6. Einrichtung nach einem der Ansprüche 1 bis 5, dadurch gekennzeichnet, daß die Wände der Flüssigkeitsbrücke voneinander einen Abstand zwischen 0,1 mm und 1 mm aufweisen.

## Claims

1. Device for removing sperm cells from seminal fluid (8), comprising a first vessel (2) for receiving the seminal fluid (8) and a second vessel (1) for receiving the sperm cells which are separated from the seminal fluid (8), wherein the two vessels are connected by a fluid bridge when the seminal fluid (8) is covered over with nutrient solution, characterised in that the cross-section of the fluid bridge is defined by fixed walls, wherein the second vessel (1) is arranged within the first vessel (2) and there is provided a bridge portion (4) which engages in a U-shape over the edge of the second vessel (1) and has an inner part (5, 5') and an outer part (6) which project into the second vessel (1) and into the first vessel (2) respectively.

2. A device according to claim 1 characterised in that the inner part (5) of the bridge portion (4) is longer than the outer part (6) which projects into the first vessel (2).

3. A device according to claim 1 or claim 2 characterised in that the inner part (5') of the bridge portion (4') is composed of a section (5'a) projecting almost to the bottom of the second vessel (1) and an inwardly adjoining section (5'b) which delimits a volume which is smaller in comparison with the volume of the second vessel (1), for receiving nutrient solution (9).

4. A device according to one of claims 1 to 3 characterised in that the wall of the first vessel (2'') that is remote from the second vessel (1) has a part (12) which slopes in the direction towards the second vessel (1), wherein a space which tapers upwardly in a direction towards the fluid bridge is formed for receiving the seminal fluid.

5. A device according to one of claims 1 to 3 characterised in that a glass portion (10) which can be fitted into the first vessel (2) forms a space tapering upwardly in a direction towards the fluid bridge, for receiving the seminal fluid (8).

6. A device according to one of claims 1 to 5 characterised in that the walls of the fluid bridge are at a spacing of between 0.1 mm and 1 mm from each other.

## Revendications

1. Dispositif pour obtenir des spermatozoïdes contenus dans du liquide séminal (8), avec un premier récipient (2) pour recevoir le liquide séminal (8) et un second récipient (1) pour recevoir les spermatozoïdes séparés du liquide séminal (8), les deux récipients étant reliés par un pont de liquide lorsque le liquide séminal (8) est recouvert d'une solution nutritive, caractérisé en ce que la section du pont de liquide est délimitée par des parois solides, le second récipient (1) étant disposé à l'intérieur du premier récipient (2) et une partie pont (4) étant prévue, s'étendant en forme de U par dessus le bord du second récipient (1) et possédant un segment interne (5,5') ainsi qu'un segment externe (6), qui dépasse dans le second (1) ou selon les cas dans le premier récipient (2).

2. Dispositif selon la revendication 1, caractérisé en ce que le segment interne (5) de la partie pont (4) est plus long que le segment externe (6) dépassant dans le premier récipient (2).

3. Dispositif selon la revendication 1 ou la revendication 2, caractérisé en ce que le segment interne (5') de la partie pont (4') se compose d'un segment partiel (5'a) dépassant presque jusqu'au fond du second récipient (1) et d'un segment partiel interne qui lui fait suite (5'b), qui délimite par comparaison avec le volume du second récipient (1) un plus faible volume pour recevoir la solution nutritive (9).

4. Dispositif selon l'une des revendications 1 à 3, caractérisé en ce que la paroi du premier récipient (2'') éloignée du second récipient (1) présente une section (12) inclinée en direction du second récipient (1), avec formation d'un espace se rétrécissant vers le haut dans la direction du pont de liquide pour recevoir le liquide séminal.

5. Dispositif selon l'une des revendications 1 à 3, caractérisé en ce qu'une partie en verre (10) pouvant être introduite dans le premier récipient (2) forme un espace se rétrécissant vers le haut en direction du pont de liquide pour recevoir le liquide séminal (8).

6. Dispositif selon l'une des revendications 1 à 5, caractérisé en ce que les parois du pont de liquide présentent entre elles un intervalle compris entre 0,1 mm et 1 mm.
